# FASCICULE DE BREVET EUROPEEN

(11) **EP 1 438 280 B1**
(45) Date de publication et mention de la délivrance du brevet: **02.01.2008**
(21) Numéro de dépôt: 02796823.9
(22) Date de dépôt: 23.10.2002
(51) Int. Cl.: C07C 43/13, C07C 49/323, C07C 69/14, C07C 69/24, C07D 317/12, C07C 13/40, C07C 49/21, C11B 9/00

(54) **NOUVEAUX DERIVES DU NORBORNANE ET DU NORBORNENE, LEUR UTILISATION ET PRODUITS PARFUMES LES CONTENANT**
NORBORNAN- UND NORBORNENDERIVATE, IHRE VERWENDUNG UND DIESE ENTHALTENDE DUFTSTOFF-PRODUKTE
NOVEL NORBORNANE AND NORBORNENE DERIVATIVES, USE THEREOF AND PERFUMED PRODUCTS CONTAINING SAME.

(30) Priorité: 23.10.2001 FR 0113663
(43) Date de publication de la demande: 21.07.2004
(73) Titulaire: V. Mane Fils S.A., 06620 Le Bar-sur-Loup (FR)
(72) Inventeur: MANE, Jean, F-06130 Grasse (FR); CHANOT, Jean-Jacques, F-06250 Mougins (FR); LE BORGNE, Fabrice, F-06330 Roquefort-Les-Pins (FR); SCHROEDER, Martin, F-06130 Grasse (FR)
(74) Mandataire: Cabinet Plasseraud
(86) Numéro de dépôt international: PCT/FR2002/003629
(87) Numéro de publication internationale: WO 2003/035595

(56) Documents cités:
- DE-A- 4 124 886
- FR-A- 1 381 663
- US-A- 4 229 600
- W.L. FANTA, ET AL.: "Studies related to the synthesis of (+/-)-dihydro-beta-santalol" JOURNAL OF ORGANIC CHEMISTRY, vol. 37, no. 10, 19 mai 1972 (1972-05-19), pages 1624-1630, XP002207436 American Chemical Society, Washington, DC, US ISSN: 0022-3263

## Description

La présente invention concerne de nouveaux dérivés du norbornane ou du norbornène, qui présentent une fragrance particulière, notamment une odeur boisée ou ambrée, et leur utilisation en parfumerie.

Le terme parfumerie est ici utilisé pour désigner non seulement la parfumerie au sens strict du terme, mais également les autres domaines dans lesquels l'odeur des produits est importante, en particulier les cosmétiques, les produits d'entretien, les désodorisants d'ambiance et autres.

Depuis longtemps, on a cherché à synthétiser de nouveaux composés ayant une odeur donnée, pour utilisation en parfumerie. On s'est souvent inspiré de produits naturels, dont on a essayé de séparer la ou les molécules présentant des propriétés olfactives.

En particulier, l'odeur d'huile de bois de santal a fait l'objet de nombreuses recherches. Après avoir identifié les deux principales molécules donnant son odeur à l'huile de bois de santal naturelle, à savoir l'α-santalol et le β-santalol, on a cherché à découvrir les autres molécules pouvant contribuer à cette odeur. On a également cherché à synthétiser des molécules pouvant reproduire cette odeur. L'historique de ces recherches est repris dans l'article « The chemistry of sandalwood fragrance - A review of the last 10 years » de E.-J. Brunke et al., 15èmes Journées Int. Huiles Essentielles, Dignes-les-Bains, France, 5-7 septembre 1996.

Le brevet US 4,229,600 a pour objet des dérivés particuliers du norbornane ou du norbornène ayant l'odeur d'huile de bois de santal.

En cherchant de nouvelles molécules, on s'est bien sûr inspiré des structures de molécules connues, mais l'expérience a montré que le résultat était souvent aléatoire. On peut par exemple citer l'article mentionné ci-dessus dans lequel est exposé un modèle de corrélation structure/odeur, immédiatement suivi d'un contre-exemple, une molécule synthétisée suivant ce modèle mais ne présentant pas l'odeur attendue. Ce même article cite un certain nombre de molécules à structure proche mais dont les propriétés olfactives sont différentes, certaines ayant une odeur, d'autres pas.

Ainsi, il n'existe pas de méthode systématique permettant de concevoir une molécule en fonction de l'odeur que l'on recherche, à partir de molécules connues, par la mise en oeuvre d'étapes logiques et reproductibles.

La Société demanderesse, après de longues recherches, a découvert une nouvelle famille de molécules odorantes.

Cette famille est représentée par la formule suivante : 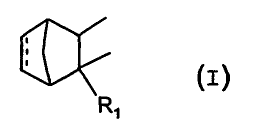 dans laquelle la liaison en pointillé est présente ou non, et dans laquelle R₁ représente :
- lorsque la liaison en pointillé est présente -CHCH₃OH ou -CHCH₃OCOR ou -CHCH₃XCH₂CHOHR' ou -CHCH₃OCHR' CH₂OH ou 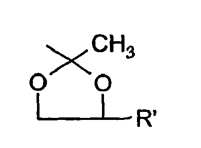
- lorsque la liaison en pointillé est absente -CHCH₃OH ou -CHCH₃OCOR ou -COCH₃ ou 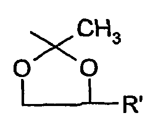 ou -CHCH₃XCH₂CHOHR' ou -CH₂CH₂XCH₂CHOHR' ou -CHCH₃OCHR' CH₂OH ou -CHCHCOR' ou -CH₂CH₂CHR'OH ou -CH₂CH₂CHR'OCOR ou -CHCHCHOHR' ou -CHCHCHR'OCOR,
dans lesquelles
R représente H, Me, Et, Pr, isoPr, But, isoBut, CH₃(CH₂)₄, (CH₃)₂CHCH₂, CH₂CH, (CH₃)₂CCH,
R' représente H, Me ou Et, et X représente O, N ou S.

Chacun des composés de cette famille peut être synthétisé directement ou indirectement à partir du composé (c) suivant :

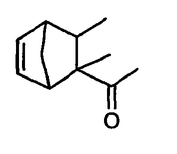

Ce composé, la 1-(2,3-diméthylbicyclo[2,2,1]-hept-5-èn)-éthanone, déjà décrit dans la littérature, et désigné dans cette demande par le terme Présantone, peut être obtenu par exemple par condensation de la méthyl-3-pentèn-3-one-2 (a) et du cyclopentadiène (b), dans une réaction de Diels-Alder en phase aqueuse catalysée par le méthylrhénium trioxyde.

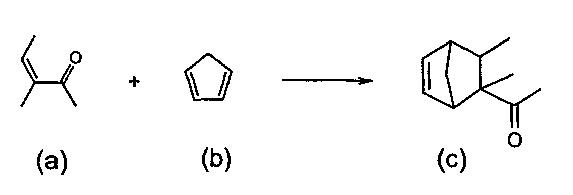

Bien entendu, d'autres réactions de préparation de la Présantone peuvent être utilisées, par exemple avec d'autres catalyseurs, ou un solvant particulier.

La méthyl-3-pentèn-3-one-2 (a) est obtenue facilement, par exemple par condensation de l'acétaldéhyde ou du paraldéhyde sur la méthyléthylcétone. Ce produit se trouve dans le commerce à bas prix.

Le cyclopentadiène (b) peut être obtenu par exemple par plusieurs méthodes connues de dépolymérisation du dicyclopentadiène produit par l'industrie du pétrole.

La Présantone peut être le produit de départ ou un intermédiaire dans la synthèse des composés selon l'invention. A titre d'exemple non limitatif, un schéma de réactions de préparation de composés nouveaux selon l'invention est donné ci-après.

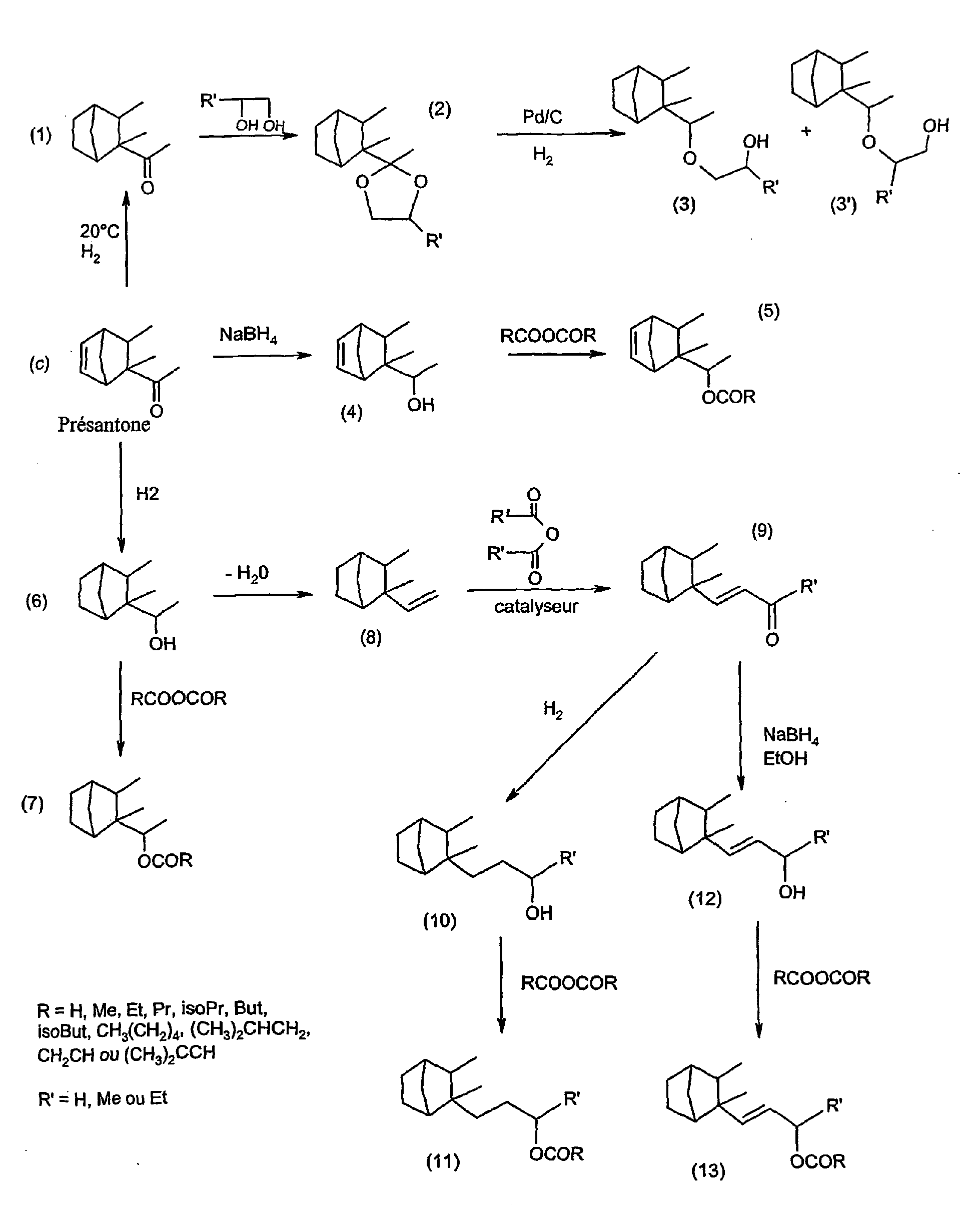

Les abréviations Me, Et, Pr, isoPr, But, isoBut ont les significations habituelles connues de l'homme du métier, à savoir méthyl, éthyl, propyl, isopropyl, butyl, isobutyl, respectivement.

L'hydrogénation de la Présantone (c) à basse température (entre 20°C et 50°C) en présence de Nickel de Raney, de palladium sur charbon, ou d'autres catalyseurs appropriés, donne la cétone saturée (1). Cette cétone possède une note boisée résineux, puissante, et dont l'originalité est appréciée des parfumeurs.

Selon l'invention, l'acétal (2) de la cétone (1) avec, par exemple, le propylène glycol, obtenu par les méthodes classiques d'acétalisation, est hydrogéné en présence, par exemple, de palladium sur charbon pur ou en mélange pour ouvrir l'acétal en alcools éthers (3) et (3'), séparables par chromatographie. L'opération est faite avec ou sans solvant. On opère de préférence sous 30 kg à 120 kg d'hydrogène à des températures comprises entre 120°C et 180°C. Les alcools éthers (3) et (3') obtenus ont une odeur boisée, puissante et tenace, d'où leur grand intérêt pour la parfumerie, les cosmétiques, les savons, les produits d'entretien, les lessives et autres produits parfumés. L'acétal (2) est lui aussi un composé nouveau intéressant en raison de son odeur.

La réduction au borohydrure de sodium de la Présantone (c) donne l'alcool insaturé (4), produit nouveau également intéressant.

Les méthodes classiques d'estérification, par exemple en utilisant les anhydrides d'acides, permettent d'obtenir les esters (5), c'est-à-dire notamment les esters formique, acétique, propionique, butyrique, isobutyrique, etc., à partir de l'alcool (4). Ces esters sont tous des composés nouveaux utilisables en parfumerie pour leur originalité dans les notes boisées.

L'hydrogénation de la Présantone (c) en présence de Nickel de Raney, par exemple, à des températures comprises entre 100°C et 180°C, sous 20 kg à 100 kg d'hydrogène, donne l'alcool saturé (6), composé également intéressant.

L'estérification de cet alcool saturé (6) par les anhydrides d'acide, par exemple, donne les esters saturés (7), R étant le radical hydrogène, méthyle, éthyle, propyle, isopropyle, butyle, isobutyle, CH₃(CH₂)₄, (CH₃)₂CHCH₂, CH₂CH, ou (CH₃)₂CCH. Tous ces nouveaux esters présentent des odeurs intéressantes pour la parfumerie, par leur caractère boisé fruité original.

Une deuxième voie a été trouvée à partir de l'alcool saturé (6). Par déshydratation de cet alcool par des méthodes bien connues, on obtient l'hydrocarbure éthylénique (8).

L'acylation, par exemple avec les anhydrides acétique ou propionique en présence d'éthérate de trifluorure de bore ou de chlorure de zinc, donne les cétones éthyléniques (9). On peut utiliser toute autre méthode connue d'acylation.

La réduction des cétones éthyléniques (9) au borohydrure de sodium, dans l'alcool, est une méthode simple pour obtenir les alcools éthyléniques (12), composés nouveaux également très intéressants. Les esters (13) des alcools (12), obtenus par les méthodes classiques, par exemple en utilisant les anhydrides d'acide, développent des notes boisées plus douces mais tenaces qui sont, elles aussi, très appréciées des parfumeurs.

L'hydrogénation totale des cétones éthyléniques (9) par les méthodes classiques donne les alcools saturés (10) et leur estérification par toute méthode usuelle, par exemple en utilisant les anhydrides d'acide, donne les esters (11), R étant le radical hydrogène, méthyle, éthyle, propyle, isopropyle, butyle, isobutyle, CH₃(CH₂)₄, (CH₃)₂CHCH₂, CH₂CH ou (CH₃)₂CCH. Ces composés présentent également des odeurs boisées intéressantes.

On peut également préparer les composés (14) et (14') -séparables par chromatographie- par ouverture de l'oxyde d'éthylène, propylène ou butylène, en éthylène glycol, propylène glycol ou butylène glycol, respectivement, avec l'alcoolate du composé (4) :

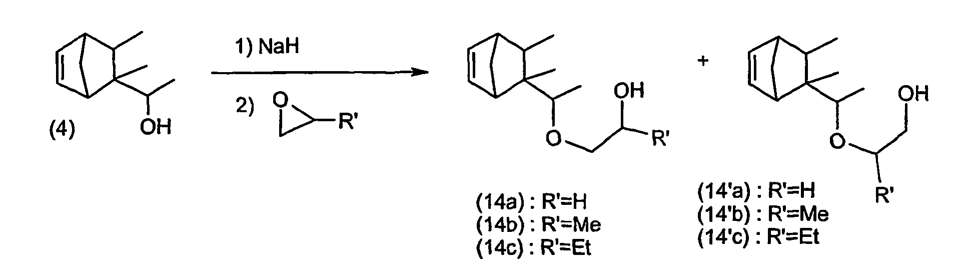

On peut citer un autre procédé de préparation des composés (3) et (3') à partir du composé (6). Ces composés peuvent être obtenus à partir de l'alcoolate du composé (6) par ouverture des oxydes d'éthylène, propylène ou butylène en glycol correspondant:

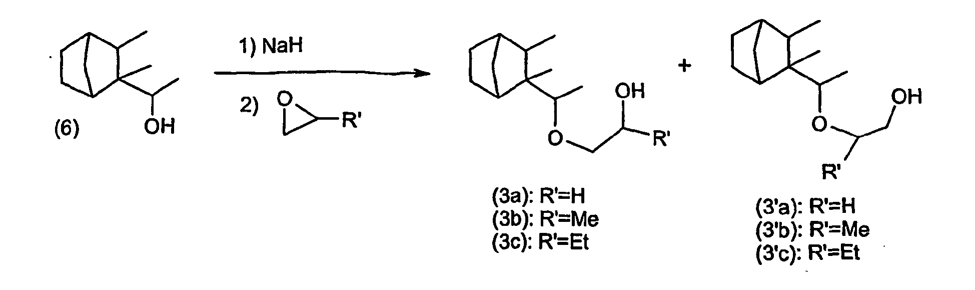

L'invention concerne également un autre procédé de préparation des composés (3) et (3'), caractérisé en ce que le produit de départ ou un intermédiaire est le composé nouveau de formule 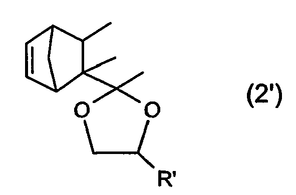 dans laquelle R' est H, Me ou Et.

L'acétal (2') peut également être le produit de départ ou l'intermédiaire dans la synthèse des composés de formule (14) et (14').

L'acétal (2') peut être préparé par exemple directement par réaction de Diels-Alder catalysée par FeCl₃ entre le cyclopentadiène et l'acétal (d).

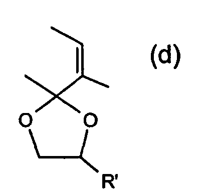

L'acétal (d) (où R' = CH3) peut être obtenu par acétalisation à partir de la méthyl-pentènone (a) et du propylène glycol.

Dans un mode de réalisation, l'acétal (2') permet la synthèse des composés (3) et (3'), par exemple par hydrogénation en une étape catalysée par le palladium sur charbon.

L'acétal (2') permet également la synthèse des composés (14) et (14') par hydrogénation, par exemple par un hydrure.

L'invention concerne également d'autres composés nouveaux, similaires aux composés (3) et (14), dans lesquels on aurait remplacé l'atome d'oxygène par un atome de soufre ou d'azote. Il s'agit des composés (15) à (18) suivants :

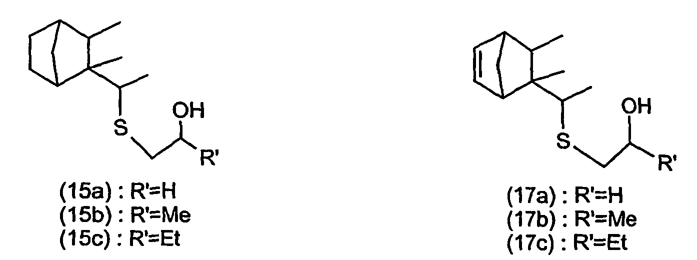

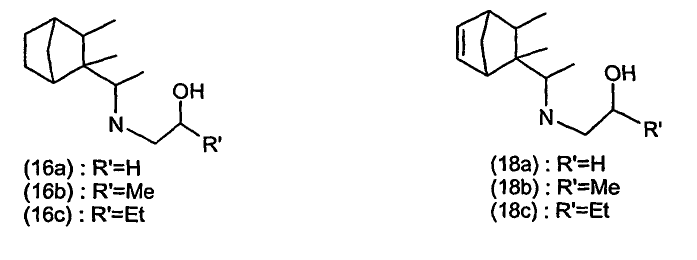

Les composés soufrés (15) [(15a), (15b) et (15c)] peuvent être obtenus à partir du composé (1) selon le procédé ci-dessous :

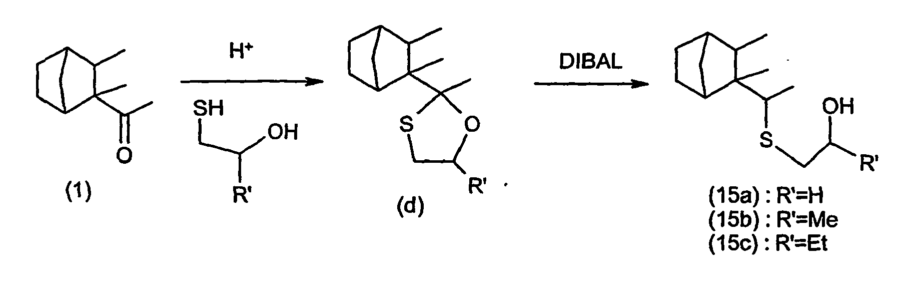

L'oxathiolane (d) peut être obtenu par une méthode classique d'acétalisation, puis l'acétal est ouvert de manière régio-spécifique par des méthodes décrites dans la littérature, par exemple avec du DIBAL à reflux du toluène.

Les composés soufrés (17) [(17a), (17b) et (17c)] peuvent être obtenus de la même façon que ci-dessus à partir de la Présantone (c).

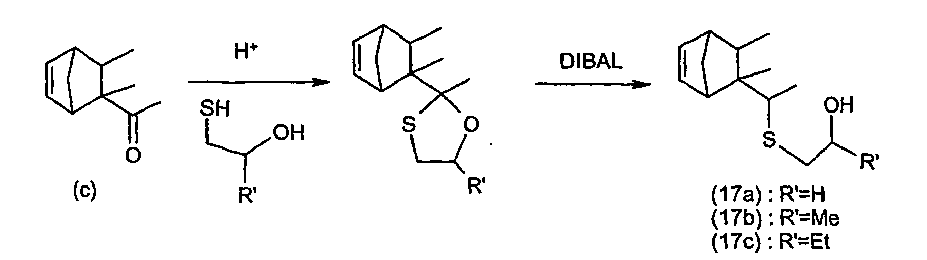

Les composés azotés (16) [(16a), (16b) et (16c)], ainsi que les composés soufrés (15) [(15a), (15b) et (15c)], peuvent être obtenus à partir de l'alcool (6) par exemple par mésylation, tosylation ou halogénation puis substitution nucléophile avec l'amino-alcool ou le thiol correspondant :

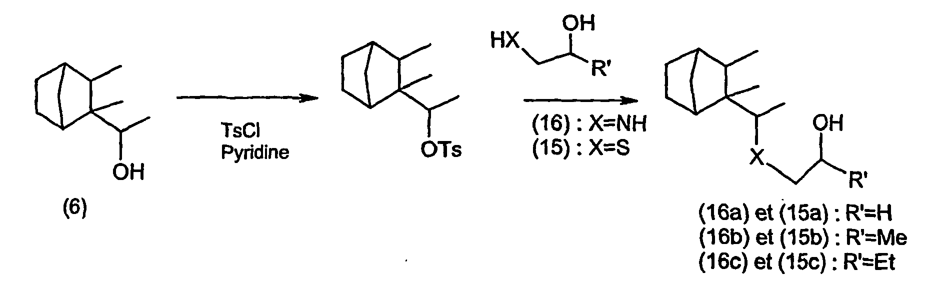

Les amino-alcools (18) [(18a), (18b) et (18c)] ainsi que les thioéthers (17) [(17a), (17b) et (17c)] peuvent être obtenus de la même manière que ci-dessus à partir de l'alcool (4). L'hydrocarbure éthylénique (8) permet aussi de synthétiser des analogues possédant un hétéroatome dans la chaîne latérale :

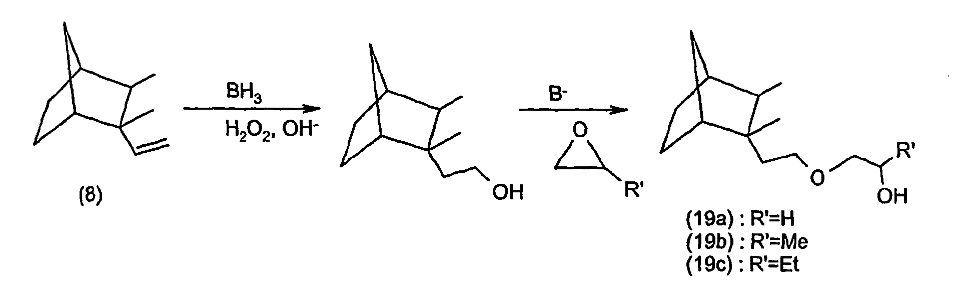

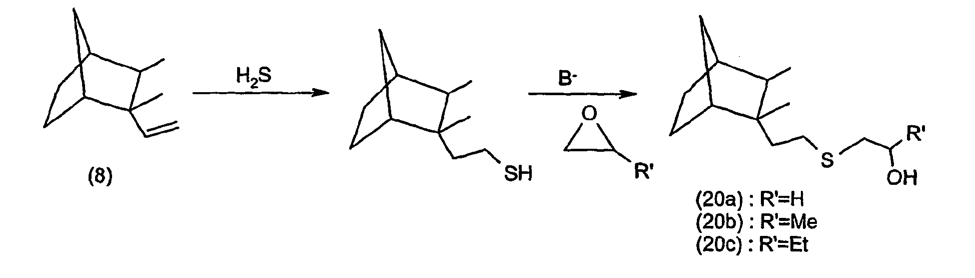

L'alcool ou le thiol primaire obtenu par hydroboration de la double liaison ou addition de H₂S est ensuite condensé sur l'oxyde d'éthylène, de propylène ou de butylène pour donner les composés (19) [a à c] et (20) [a à c]. L'oxyde de propylène peut éventuellement être remplacé par l'halogéno-acétone correspondante pour donner après réduction au NaBH₄ les alcools (19b) et (20b) attendus.

Les analogues aminés (21) [a à c] peuvent être obtenus à partir de l'alcool primaire issu de l'hydrogénation par tosylation (ou mésylation) suivie d'une substitution avec l'amino alcool correspondant :

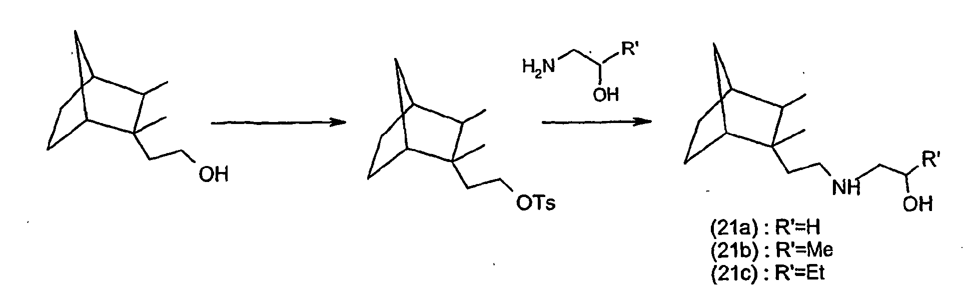

L'invention a pour objet les composés représentés par la formule générale (I) sous forme de mélange de diastéréoisomères en proportions variables, en particulier les mélanges racémiques.

Par ailleurs, certains des diastéréo-isomères ou énantiomères possèdent des propriétés olfactives aussi intéressantes que les composés obtenus sous forme de mélanges diastéréo-isomériques.

L'invention a également pour objet les diastéréoisomères ou énantiomères purs des nouveaux composés représentés par la formule générale (I), qui peuvent être séparés par chromatographie gazeuse préparative. Ils peuvent aussi être synthétisés à partir de composés optiquement actifs, par exemple le propylène glycol R ou S et l'alcool (6) provenant de la réduction énantio-sélective de la Présantone (1-4), et purifiés par distillation.

L'invention a également pour objet les procédés de préparation des nouveaux composés.

Chacun des nouveaux composés présente un intérêt en raison de son odeur, en particulier boisée. Le composé (3) avec R' = Me est particulièrement intéressant. Par ailleurs, l'invention présente un intérêt économique évident qui découle de la simplicité des réactions mises en oeuvre et du faible coût des matières premières utilisées.

De par leurs qualités olfactives, ces différents composés trouvent ainsi un emploi très varié en parfumerie pour la préparation de bases et concentrés parfumants, parfums et eaux de toilette, ainsi que dans le parfumage d'articles de consommation divers, tels que savons, gels douches ou de bain, shampooings et autres produits d'hygiène capillaire, préparations cosmétiques, désodorisants corporels ou d'air ambiant, ou encore détergents ou adoucissants textiles et produits d'entretien.

Dans ces applications, les composés selon l'invention peuvent être employés seuls ou, comme il est plus courant en parfumerie, en mélange avec d'autres ingrédients parfumants, des solvants ou des adjuvants d'usage courant en parfumerie et que l'homme du métier est à même de choisir en fonction de l'effet désiré et de la nature du produit à parfumer.

Les concentrations dans lesquelles ces composés et leurs mélanges isomériques peuvent être utilisés pour obtenir les effets parfumants désirés varient dans une gamme de valeurs très étendue (0,1% à 99 %), étant bien connu que ces valeurs dépendent de la nature de l'article à parfumer, de l'effet odorant recherché, et de la nature des autres ingrédients dans une composition donnée.

L'invention a donc aussi pour objet l'utilisation des composés nouveaux pour la préparation d'une composition parfumante ou d'un article parfumé, dans les applications décrites ci-dessus, en particulier en parfumerie, en cosmétique, par exemple pour des shampooings ou des savons, et pour des produits d'entretien, tels que des assouplissants ou des lessives.

L'invention a également pour objet les produits parfumés comprenant au moins un composé selon la présente invention, que ce soit dans le domaine de la parfumerie, de la cosmétique ou de l'entretien.

Les exemples suivants illustrent davantage les différents procédés de fabrication des composés nouveaux selon l'invention ainsi que leur utilisation, et leur intérêt. Ces exemples ne sont présentés que dans un but d'illustration et ne peuvent être considérés comme limitatifs de l'invention.

### Exemple 1 : Réaction de Diels-Alder (Présantone (c))

Dans un ballon de 500 ml avec agitation magnétique, thermomètre, ampoule à introduire et placé sous azote, on charge 110 g de toluène sec.

Après refroidissement à 0°C, on ajoute d'un coup 4 g de chlorure d'aluminium puis, vers 10°C, 88 g de méthyl-3-pentèn-3-one-2 (a), à 94 % en 20 à 30 minutes en refroidissant légèrement.

Entre 12°C et 14°C, on introduit ensuite une solution de 66 g de cyclopentadiène (b) dans 140 g de toluène sec en 1h10 à 1h20, en maintenant la température en refroidissant.

Après décantation, la phase organique est lavée à l'acide chlorhydrique dilué (100 ml) et avec une solution de carbonate à 5%. Après concentration sous vide du solvant, le produit est distillé sous 266 Pa (2mm) de vide (T_{eb} : 56-70°C 266 Pa (2mm)). On obtient 128 g de distillat. Ce produit est repris au toluène et traité à la triéthylamine et avec une solution de carbonate de sodium. Après lavage et évaporation du solvant sous vide, le produit est redistillé sous vide de 133 Pa (1mm). On obtient 112 g de 1- (2, 3-diméthyl-bicyclo[2,2,1]-hep-5-èn) -éthanone (T_{eb}: 56-60°C 133 Pa (1mm)), rendement: 76 %.

On laisse encore agiter à 14-15°C pendant 15 minutes puis on refroidit à 0°C et on ajoute rapidement une solution de 20 g d'acide chlorhydrique à 32 % dans 100 g d'eau en laissant remonter la température.

L'analyse CPV montre la présence de 2 isomères principaux (32% et 56%).

Les analyses des spectres infra rouge et de masse correspondent aux structures des composés attendus.

### Exemple 2 : Cétone saturée (1)

L'hydrogénation de 50 g de la cétone éthylénique (c) en présence de 0,5 g de Nickel de Raney à température ambiante, et sous faible pression d'hydrogène (1 à 10 kg) absorbe en 2 heures environ la quantité théorique d'hydrogène.

Après séparation du catalyseur, le produit est distillé sous vide. La cétone saturée (1) est obtenue de façon pratiquement quantitative.

Le produit distille à 54°C-58°C sous 133 Pa (1mm) de vide.

L'analyse CPV montre 2 pics principaux et les spectres infra rouge et de masse correspondent aux structures des composés attendus.

### Exemple 3 : Alcool éthylénique (4)

Par réduction de 40 g de Présantone (c) dans 200 ml d'alcool à 96 % en ajoutant 6 g de borohydrure de sodium par portions à 25°C en 2 heures, puis en portant progressivement à 40°C en 2 heures et en maintenant encore 3 heures cette température, on obtient la réduction totale de la fonction cétone en alcool.

Après acidification lente, avec de l'acide chlorhydrique à 2 %, extraction au toluène et lavages, le produit est concentré sous vide puis distillé.

On obtient 36 g d'alcool éthylénique (4), ayant une température d'ébullition de 72°-75°C sous 133 Pa (1 mm) de vide, soit 90 % de rendement molaire.

L'analyse CPV montre la présence de 3 isomères principaux qui ont des spectres de masse et infra rouge très voisins.

### Exemple 4 Esters éthyléniques (5)

En portant lentement à 1.15°-1,20°C 10 g d'alcool éthylénique (4) dans 10 g d'anhydride acétique pendant 2 heures, on obtient l'acétate éthylénique (5).

Le produit obtenu est concentré sous vide de 3990 Pa (30mm) pour distiller l'acide et l'anhydride acétique en excès à travers une colonne Vigreux de 25 cm. Le vide est amélioré lentement jusqu'à 133 Pa (1mm) et on distille l'acétate éthylénique (5).

On obtient 10 g d'acétate éthylénique (5), ayant une température d'ébullition de 73°, 76°C, sous 133 Pa (1 mm) de vide.

Les analyses CPV, SM et IR correspondent aux structures des composés attendus.

Les esters propionique, butyrique et isobutyrique de l'alcool éthylénique (4) sont obtenus en chauffant l'alcool (4) avec les anhydrides correspondants, en présence d'un peu de toluène pour maintenir la température de reflux vers 125°-130°C.

Le temps de reflux est porté à 2 heures pour terminer l'estérification.

Par distillation sous vide, on sépare les acides et les esters d'anhydrides. En améliorant le vide, on obtient les esters (5).

Propionate de l'alcool éthylénique (5), ayant une température d'ébullition de 80°-83°C, sous 133 Pa (1 mm) de vide.

Butyrate de l'alcool éthylénique (5), ayant une température d'ébullition de 88°-91°C, sous 133 Pa (1 mm) de vide.

Isobutyrate de l'alcool éthylénique (5), ayant une température d'ébullition de 85°-88°C, sous 133 Pa (1 mm) de vide.

### Exemple 5 Alcool saturé (6)

100 g de cétone éthylénique (c) sont chargés dans un autoclave agité et chauffé en présence de 2 g de Nickel de Raney.

Après 3 purges avec 10 kg d'hydrogène, la pression est portée à 30 kg et l'autoclave est chauffé à 150°, 160°C sous agitation.

L'hydrogénation est faite sous 60 kg d'hydrogène jusqu'à ce que la pression ne baisse plus (5 à 6 heures).

Le produit saturé (5) brut est distillé sous 133 Pa (1 mm) de vide dans un appareil muni d'une colonne Vigreuxde 20 cm.

On obtient 97 g d'alcool (5), ayant une température d'ébullition de 72°-75°C, sous 133 Pa (1 mm) de vide.

L'analyse CPV montre qu'on a 4 isomères principaux qui ont des spectres de masse et infra rouge très proches.

### Exemple 6 : Esters saturés (7)

Les esters saturés sont obtenus par la méthode classique utilisée pour faire les esters insaturés (Exemple 4), c'est-à-dire le chauffage de l'alcool saturé (6) en présence de l'anhydride d'acide correspondant.

Parmi les composés obtenus :
Acétate de l'alcool saturé (7),ayant une température d'ébullition de 73°-76°C, sous 133 Pa (1 mm) de vide,
Propionate de l'alcool saturé (7), ayant une température d'ébullition de 80°-83°C, sous 133 Pa (1mm) de vide,
Butyrate de l'alcool saturé (7), ayant une température d'ébullition de 88°-91°C, sous 133 Pa (1mm) de vide,
Isobutyrate de l'alcool saturé (5), ayant une température d'ébullition de 85°-88°C, sous 133 Pa (1 mm) de vide.

### Exemple 7 Ether alcool (3)

### 7.1.-Acétal (2)

L'acétal de la cétone saturée (1) et du propylène glycol peut être préparé en portant au reflux, avec colonne Vigreux de 30 cm et séparateur d'eau, le propylène glycol (50 g), la cétone (1) (80 g) en solution dans le toluène (40 g) et de l'acide p-toluène sulfonique (0,4 g) comme catalyseur acide. L'eau est éliminée par azéotropie (reflux 18 à 20 heures).

On peut aussi supprimer le toluène et éliminer l'eau en la distillant sous vide.

Le mélange réactionnel est lavé avec une solution de carbonate de soude à 5 % puis à l'eau jusqu'à pH neutre.

Le fractionnement sous bon vide dans un appareil muni d'une colonne Vigreux de 50 cm permet d'obtenir :
25 g de cétone (1) récupérée à recycler, ayant une température d'ébullition de 54°-58°C, sous 133 Pa (1 mm) de vide,
65 g Acétal (2), ayant une température d'ébullition de 64°-58°C, sous 133 Pa (1 mm) de vide,
10 g fractions intermédiaires à recycler.

### 7.2. Ether alcool (3)

Dans un autoclave agité, on charge 50 g d'acétal (2), 100 g d'isopropanol et 0,5 g de palladium sur charbon.

Après les purges à l'hydrogène, on porte 18 à 24 heures entre 160° et 170°C sous 50-60 kg d'hydrogène.

L'avancement de la réaction est suivi par CPV et on arrête quand le pourcentage d'acétal (2) est tombé à 5 à 6 %.

Après séparation du catalyseur, le produit est distillé sous 133 Pa (1 mm) de vide dans un appareil muni d'une colonne Vigreux de 50 cm. On obtient 6 g de têtes

Température d'ébullition 40°-50°C, 16 g de cétone (1) + acétal (2), ayant une température d'ébullition de 54°-68°C sous 1 mm de vide, à recycler et 25 g d'éther alcool (3), ayant une température d'ébullition de 85°-92°C sous 133 Pa (1 mm) de vide. Les spectres de masse et infra rouge correspondent aux structures des composés attendus.

### Exemple 8 : Cétone éthylénique (9)

### 8.1. Déshydratation de l'alcool (6)

Dans un ballon de 500 ml, on charge 150 g d'alcool (6) et 100 g d'acide borique.

On chauffe sous 5320 Pa (40 mm) de vide pour sortir l'eau de boratisation puis on porte à température à 230°-250°C pour déshydrater. Après avoir distillé 70 à 80 g d'éthylénique (8) on poursuit la déshydratation en introduisant l'alcool (6) au fur et à mesure de la déshydratation.

A partir de 1 kg d'alcool (6), on obtient 880 g d'éthylénique (8) contenant 7 à 10 % d'alcool (5).

Par fractionnement sur une colonne Vigreux de 30 cm, on sépare 800 g d'éthylénique (8) et on récupère 70 g d'alcool (6) à recycler.

### 8.2. Cétone éthylénique (9)

Dans un ballon de 1 litre avec agitation magnétique, on charge 120 g d'éthylénique (8) 120 g d'anhydride acétique et on chauffe le mélange à 70°-75°C. On introduit alors 16 ml d'éthérate de trifluorure de bore en 15 minutes à 75°-80°C. La température et l'agitation sont maintenues 3 heures à 80°-82°C.

Après refroidissement vers 60°C, on décompose l'anhydride acétique en excès en ajoutant, lentement au début, 320 g d'eau en maintenant la température vers 60°C-65°C.

La phase organique est séparée et la phase aqueuse est extraite avec 2 fois 60 ml de cyclohexane. Les phases organiques réunies sont lavées avec une solution de carbonate de sodium à 10 %, puis à l'eau.

Après concentration du solvant sous léger vide, le produit brut (138 g) est fractionné dans un appareil avec colonne Vigreux de 30 cm. Sous 665 Pa (5 mm) de vide on récupère 80 g de produit de départ (8) à recycler et 32 g de cétone éthylénique (9) ayant une température d'ébullition de 75°-88°C, sous 133 Pa (1 mm) de vide.

### Exemple 9 : Alcool éthylénique (12)

Dans un ballon de 250 ml avec agitation magnétique et thermomètre, on charge 30 g de cétone éthylénique (9) et 75 g d'éthanol à 96°. Sous léger refroidissement, pour maintenir 20°-25°C, on introduit en petites portions 3 g de borohydrure de sodium. Après l'introduction, on laisse encore tourner 6 heures à température ambiante puis on chauffe progressivement à 40°C en 2 à 3 heures, puis on porte à 40°-45° pendant 4 heures.

Une grande partie de l'alcool est concentrée sous léger vide. Le culot est additionné de 30 g d'eau puis acidifié lentement en maintenant 20°-25°C, avec 16 g d'acide chlorhydrique concentré dilué avec 50 g d'eau. On ajoute alors 30 ml de toluène, avant de décanter. La phase organique est séparée et les eaux sont extraites avec 2 fois 20 ml de toluène. Après réunion des phases organiques, on lave 2 fois 80 ml d'eau. Le toluène est ensuite concentré sous vide et le culot (30 g) est distillé sous bon vide dans un appareil avec colonne Vigreux de 15 cm.

On obtient ainsi 25 g d'alcool éthylénique (12), ayant une température d'ébullition de 85°-92°C, sous 133 Pa (1 mm) de vide, et 3 g de produit à reprendre. Les spectres infra rouge et de masse correspondent aux structures des composés attendus.

### Exemple 10 Acétate éthylénique (13)

Dans un ballon de 100 ml, avec agitation magnétique, thermomètre et colonne Vigreux de 20 cm, on charge 30 g d'alcool éthylénique (12) et 25 g d'anhydride acétique.

On chauffe sous léger vide de façon telle que la température dans la masse soit de 115°-120°C pendant 2 heures. Le vide est ensuite amélioré pour distiller le mélange acide/anhydride acétique. On termine à 125°-130°C dans la masse.

Sous 133 Pa (1 mm) de vide, on distille ensuite l'acétate (14) ayant une température d' ébullition de 80°-88°C, sous 133 Pa (1mm) de vide. On obtient 27 g d'acétate (13) et 3 g de produit à reprendre. Les spectres infra rouge et de masse correspondent aux structures des composés attendus.

### Exemple 11: Alcool saturé (10)

Dans un autoclave agité et chauffé, on charge 30 g de cétone éthylénique (9), 0,6 g de Nickel de Raney. Après les purges à l'hydrogène, on porte à 150°C-160°C sous 60 kg d'hydrogène.

Après 12 heures la réaction est terminée. Le catalyseur est séparé et le produit est distillé sous bon vide. On obtient 27 g d'alcool saturé (11), ayant une température d'ébullition de 82°-90°C sous 133 Pa (1mm) de vide.

### Exemple 12 : Acétate saturé (11)

En opérant comme pour l'acétate éthylénique (13) sur 25 g d'alcool saturé (10) on obtient par distillation 26 g d'acétate (11), ayant une température d'ébullition de 78°-86°C sous 133 Pa (1 mm) de vide.

### Exemple 13 : Ether alcool (3) par l'intermédiaire de l'acétal (2')

On place sous azote 46,00 g de dichlorométhane dans le ballon de réception d'un appareil de distillation surmonté d'une colonne Vigreux de 30 cm et on le refroidit à 0°C. On chauffe le bain d'huile de l'appareil de distillation à environ 195°C pour obtenir une température de 165°C à l'intérieur du tricol. On introduit via la tête de la colonne, goutte à goutte, 13,80 g (0,45 mol) de dicyclopentadiène, en récupérant le cyclopentadiène ainsi fraîchement dépolymérisé dans le solvant refroidi. On obtient en 4 heures 56,00 g d'une solution de cyclopentadiène dans le dichlorométhane, donc un potentiel de 10,00 g de cyclopentadiène (0,151 mol). Rendement : 72,5%. La solution est directement engagée dans la réaction de Diels-Alder suivante.

On introduit dans le ballon sous azote et à température ambiante 14,80 g (0,076 mol) de 2,4-diméthyl-2-(1-méthylpropényl)-[1,3]dioxalane à 80,0% et 1.5 g de fer (III) chlorure de silice, et on refroidit à 0°C. On ajoute goutte à goutte à cette température pendant 10 minutes 56,00 g de la solution de cyclopentadiène dans le dichlorométhane. On agite pendant 2 heures à 0°C, puis pendant 15 heures en laissant la température dans la masse remonter à température ambiante. On filtre le catalyseur et on évapore le solvant sous pression réduite (environ 35 mm Hg = 4,67 10³ Pa). On obtient 23,00 g d'une huile jaune clair, qui sont transférés dans un appareil de micro-distillation avec une colonne Vigreux de 15 cm. On obtient 1,5 g (5,6 mmol) de 2-(2,3-diméthyl-bicyclo[2.2.1]hept-5-ên-2-yl)2,4-diméthyl-[1,3]dioxalane à 83% (somme des isomères). Rendement : 7,4%.

### Exemple 14 : évaluation olfactive

Dans un premier temps, les caractéristiques olfactives du composé (3) quand R' est le méthyl ont été évaluées par un panel en même temps que les caractéristiques olfactives de composés connus présents sur le marché. Le panel d'évaluation est composé de plusieurs professionnels, évaluant qualitativement et quantitativement chaque composé. Les résultats des évaluations sont rassemblés dans le tableau ci-après.

| Odeur : | Boisé | Cèdre | Ambré | Vetyver | Irisé | Camphré | Cuir |
|---|---|---|---|---|---|---|---|
| Composé (3) | XXX | XX | XX | | | XX | |
| Iso E Super⁽¹⁾ | XXX | X | XXX | X | | | |
| Vertofix⁽²⁾ | XXX | XX | | | XX | | X |
| Boisambrene⁽³⁾ | XXX | | XX | | | | X |

| | | | | | | | |
|---|---|---|---|---|---|---|---|
| ⁽¹⁾ 7-Acétyl-1,2,3,4, 5,6,7,8-octahydro-1,1,6,7-tétra méthyl-naphtalène (mélange d'isomères) ; origine : International Flavours & Fragrances , USA. ⁽²⁾ 1-(3,6,8,8,-tetraméthyl-2,3,4,7,8,8a,hexahydro,1H,3a,7-méthanoazulen-5-yl)-éthanone ; origine : International Flavours & Fragrances, USA. ⁽³⁾ (Ethoxyméthoxy)-cyclododécane ; origine : HENKEL. | | | | | | | |

Ces trois produits de référence sont très largement utilisés dans les compositions parfumantes pour savons, détergents et assouplissants ou shampooings. Ils sont aussi très utilisés en parfumerie alcoolique.

On peut observer que le composé (3) présente un caractère boisé, de cèdre, ambré et camphré.

On a dans un deuxième temps évalué l'impact ou les caractéristiques olfactives du composé (3) lorsqu'il est appliqué sur un support.

Trois supports différents ont été utilisés:
- en solution à 10% dans l'alcool 96°,
- dans une base assouplissant standard à 0,5% : évaluation sur linge humide et sur linge sec,
- dans une base shampooing standard à 0,5% :

évaluation de la couverture de base et diffusion en solution dans l'eau chaude.

Deux compositions parfumantes de base ont été préparées en mélangeant les ingrédients listés ci-après. Les essais ont été réalisés en ajoutant 50 parties en poids de composé (3) au mélange de base.

| COMPOSITION N° 1 Essai dans détergent (application à 0,5 %) | | |
|---|---|---|
| COMPOSANTS | ESSAI 1 | ESSAI 2 |
| GERANIUM RECO VMF⁽¹⁾ | 20 | 20 |
| BENZYLE ACETATE | 60 | 60 |
| DIMETHYLBENZYLCARBINYLE ACETATE | 20 | 20 |
| PHENOXYALLYLE ACETATE | 2 | 2 |
| PHENYLETHYLIQUE ALCOOL | 100 | 100 |
| HEXYLCINNAMIQUE ALDEHYDE | 200 | 200 |
| ALDEHYDE C12 LAURIQUE | 2 | 2 |
| ALDEHYDE C12 MNA | 3 | 3 |
| GAMMA UNDECALACTONE | 7 | 7 |
| CITRONELLOL | 30 | 30 |
| COUMARINE | 15 | 15 |
| DIHYDROFLORIFONNE ⁽²⁾ | 1 | 1 |
| DIHYDROMYRCENOL | 50 | 50 |
| VERDYLE ACETATE⁽³⁾ | 15 | 15 |
| GIROFLE MADAGASCAR ESS. | 12 | 12 |
| LILIAL⁽⁴⁾ | 70 | 70 |
| METHYLIONONE | 55 | 55 |
| PHENYLE OXIDE | 25 | 25 |
| ROSE OXIDE | 3 | 3 |
| AMYLE SALICYLATE | 95 | 95 |
| BENZYLE SALICYLATE | 135 | 135 |
| TRIPLAL⁽⁵⁾ | 4 | 4 |
| VERDOX⁽⁶⁾ | 20 | 20 |
| OXYPHENYLON à 10 % EDG | 6 | 6 |
| COMPOSE (3) | | 50 |
| | | |
| TOTAL | 950 | 1000 |

| | | |
|---|---|---|
| ⁽¹⁾ V. MANE FILS ⁽²⁾ 1-(2,6,6-triméthyl-3-cyclohexèn-1-yl)-2-butèn-1-one ⁽³⁾ 3a,4,5,6,7,7a-hexahydro-4,7-méthano-1H-inden-5 (ou 6)-ol acétate - origine GIVAUDAN (Suisse) ⁽⁴⁾ p-tert-butyl-alpha-méthyl-hydrocinnamaldéhyde - origine GIVAUDAN (Suisse) ⁽⁵⁾ 2,4-diméthyl-3-cyclohexène-1-carbaldéhyde - origine IFF (International Flavours and Fagrances, Etats-Unis) ⁽⁶⁾ 2-tert-butylcyclohexyl acétate - origine IFF | | |

| COMPOSITION N° 2 Essai dans parfum (application à 7 %) | | |
|---|---|---|
| COMPOSANTS | ESSAI 1 | ESSAI 2 |
| STYRAX RECO VMF⁽¹⁾ | 5 | 5 |
| ISOBORNYLCYCLOHEXANOL à 50% EDG | 90 | 90 |
| YLANG RECO⁽¹⁾ | 25 | 25 |
| BENZYLE ACETATE | 80 | 80 |
| PHENYLETHYLIQUE ALCOOL | 80 | 80 |
| ANISIQUE ALDEHYDE | 10 | 10 |
| BACDANOL⁽²⁾ | 20 | 20 |
| CITRONELLOL | 55 | 55 |
| COUMARINE | 35 | 35 |
| DIHYDROMYRCENOL | 20 | 20 |
| ETHYL VANILLINE | 7 | 7 |
| GALAXOLIDE⁽³⁾ | 140 | 140 |
| GERANIOL | 80 | 80 |
| HELIOTROPINE | 10 | 10 |
| IRISONE PURE⁽⁴⁾ | 25 | 25 |
| LILIAL | 45 | 45 |
| METHYLIONONE | 25 | 25 |
| BENZYLE SALICYLATE | 80 | 80 |
| VANILLINE | 8 | 8 |
| VERTENEX⁽⁵⁾ | 80 | 80 |
| ALDEHYDE C10 CAPRIQUE à 10% DPG | 10 | 10 |
| ALDEHYDE C12 LAURIQUE à 10 % DPG | 15 | 15 |
| INDOL à 10 % DPG | 5 | 5 |
| COMPOSE (3) | | 50 |
| | | |
| TOTAL | 950 | 1000 |

| | | |
|---|---|---|
| ⁽¹⁾ V. MANE FILS ⁽²⁾ 4-(2,2,3-triméthyl-3-cyclopentényl)-2-éthyl-3-butèn-1-ol - origine IFF ⁽³⁾ 1,3,4,6,7,8-hexahydro-4,6,6,7,8,8-hexaméthylcyclopenta-(g)-2-benzan - origine IFF ⁽⁴⁾ Mélange d'ionones ⁽⁵⁾ 4-tert-butylcyclohexyl acétate - origine IFF | | |

Dans chaque cas, les évaluations de l'impact olfactif ont été effectuées à t₀, t₊₄₈ₕ, t₊₁₆₈ₕ pour évaluer les notes de tête, de coeur et de fond.

Dans tous les cas, on observe que le composé (3) apporte une note de tête et une couverture de base intéressantes avec un caractère boisé, camphré riche et original.

Que ce soit en solution alcoolique (composition 2) ou en assouplissant (composition 1), la note boisée ambrée riche est très perceptible après 48h de séchage et apporte une touche originale propre et cosmétique à la fois.

La perte d'intensité avec le temps semble assez linéaire sans laisser apparaître un important changement de caractéristique olfactive.

La diffusion dans l'eau chaude en shampooing affiche un caractère boisé discret mais sophistiqué et très agréable.

Les résultats de ces évaluations montrent sans le moindre doute que le composé (3) présente des caractéristiques olfactives intéressantes, qui trouveront une application en particulier dans les cosmétiques, la parfumerie, les produits d'entretien.

## Revendications

1. Nouveau composé de formule : 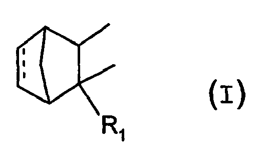 dans laquelle la liaison en pointillé est présente ou non, et dans laquelle R₁ représente :
- lorsque la liaison en pointillé est présente -CHCH₃OH ou -CHCH₃OCOR ou -CHCH₃XCH₂CHOHR' ou -CHCH₃OCHR' CH₂OH ou 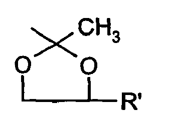
- lorsque la liaison en pointillé est absente 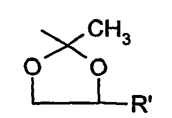 -CHCH₃OH ou -CHCH₃OCOR ou -COCH₃ ou -CHCH₃XCH₂CHOHR' ou -CH₂CH₂XCH₂CHOHR' ou -CHCH₃OCHR'CH₂OH ou -CHCHCOR' ou -CH₂CH₂CHR'OH ou -CH₂CH₂CHR'OCOR ou -CHCHCHOHR' ou -CHCHCHR'OCOR,
dans lesquelles
R représente H, Me, Et, Pr, isoPr, But, isoBut, CH₃(CH₂)₄. (CH₃)₂CHCH₂, CH₂CH, (CH₃)₂CCH,
R' représente H, Me ou Et, et X représente O, N ou S.

2. Composé selon la revendication 1, **caractérisé en ce que** la liaison en pointillés est absente, et R₁ représente -CHCH₃OCH₂CHOHCH₃ ou -CHCH₃OCHR'CH₂OH.

3. Composé selon l'une ou l'autre des revendications 1 et 2, sous forme d'un diastéréoisomère ou énantiomère.

4. Composé selon l'une ou l'autre des revendications 1 et 2, sous forme d'un mélange de diastéréoisomères.

5. Composé selon l'une ou l'autre des revendications 1 et 2, sous forme d'un mélange racémique.

6. Procédé de préparation d'un composé selon l'une quelconque des revendications 1 à 5, **caractérisé en ce que** le produit de départ ou un intermédiaire est le composé de formule :

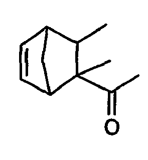

7. Procédé de préparation d'un composé selon l'une quelconque des revendications 1 à 5 de formule (I) dans laquelle R1 est -CHCH₃OCH₂CHOHR' ou -CHCH₃OCHR' CH₂OH et R' est H, Me ou Et, **caractérisé en ce que** le produit de départ ou un intermédiaire est le composé de formule

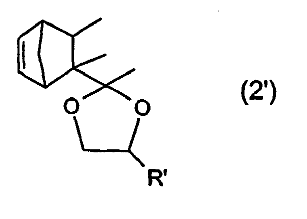

8. Utilisation d'un composé selon l'une quelconque des revendications 1 à 5 à titre d'ingrédient parfumant pour la préparation d'une composition parfumante ou d'un article parfumé.

9. Produit parfumé, notamment parfum et eau de toilette, cosmétique tels que savon, gel douche ou de bain, shampooing et autre produit d'hygiène capillaire, produit d'entretien, lessive et assouplissant, désodorisant corporel ou d'air ambiant, **caractérisé en ce qu'**il comprend un composé selon l'une quelconque des revendications 1 à 5 ou obtenu par le procédé selon l'une quelconque des revendications 6 et 7.

## Claims

1. A novel compound of formula: 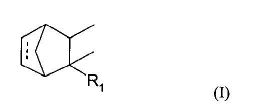 wherein the dotted bond is present or absent, and wherein R₁ represents:
- when the dotted bond is present:
-CHCH₃OH or -CHCH₃OCOR or -CHCH₃XCH₂CHOHR' or -CHCH₃OCHR'CH₂OH or 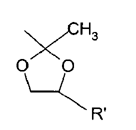
- when the dotted bond is absent 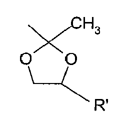 or -CHCH₃OH or -CHCH₃OCOR or -COCH₃ or -CHCH₃XCH₂CHOHR' or -CH₂CH₂XCH₂CHOHR' or -CHCH₃OCHR'CH₂OH or -CHCHCOR' or -CH₂CH₂CHR'OH or -CH₂CH₂CHR'OCOR or -CHCHCHOHR' or -CHCHCHR'OCOR, wherein R represents H, Me, Et, Pr, isoPr, But, isoBut, CH₃(CH₂)₄, (CH₃)₂CHCH₂, CH₂CH or (CH₃)₂CCH, R' represents H, Me or Et, and X represents O, N or S.

2. The compound as claimed in claim 1, wherein the dotted bond is absent and R₁ is -CHCH₃OCH₂CHOHCH₃ or -CHCH₃OCHR'CH₂OH.

3. The compound as claimed in anyone of claims 1 and 2, in the form of either a diastereoisomer or an enantiomer.

4. The compound as claimed in anyone of claims I and 2, in the form of a mixture of diastereoisomers.

5. The compound as claimed in anyone of claims I and 2, in the form of a racemic mixture.

6. A process for preparing a compound as claimed in anyone of claims 1 to 5 wherein the starting compound or an intermediary compound is of formula:

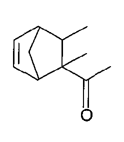

7. A process for preparing a compound as claimed in anyone of claims 1 to 5, of formula (I) wherein R1 represents -CHCH₃OCH₂CHOHR' or -CHCH₃OCHR'CH₂OH and R' represents H, Me or Et, wherein the starting compound or an intermediary compound is of formula:

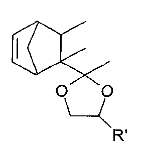

8. Use of a compound as claimed in anyone of claims 1 to 5, as fragrant ingredient for the preparation of a fragrant composition or a fragrant article.

9. Fragrant product, including perfumes and eaux de toilette, cosmetics such as soaps, shower gels or bath gels, shampoos and other hair hygiene products, cleaning products, textile detergents or softeners, air fresheners, deodorants, including a compound according to anyone of claims 1 to 5 or obtainable by the process according to anyone of claim 6 or 7.

## Patentansprüche

1. Neue Verbindung der Struktur: 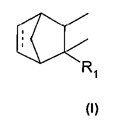 in welcher die gestrichelt gezeichnete Bindung anwesend ist oder nicht, und in denen R₁ für,
- wenn die gestrichelt gezeichnete Bindung anwesend ist:
-CHCH₃OH oder -CHCH₃OCOR oder -CHCH₃XCH₂CHOHR' oder -CHCH₃OCHR'CH₂OH, 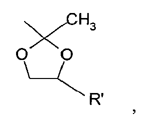
- wenn die gestrichelt gezeichnete Bindung abwesend ist: 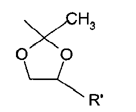 -CHCH₃OH oder -CHCH₃OCOR oder -COCH₃ oder -CHCH₃XCH₂CHOHR' oder -CH₂CH₂XCH₂CHOHR' oder -CHCH₃OCHR'CH₂OH oder -CHCHCOR' oder -CH₂CH₂CHR'OH oder -CH₂CH₂CHR'OCOR oder -CHCHCHOHR' oder CHCHR'OCOR steht,
in welchen
R für H, Me, Et, Pr, isoPr, But, isoBut, CH₃(CH₂)₄, (CH₃)₂CHCH₂, CH₂CH oder (CH₃)₂CCH steht,
R' für H, Me oder Et steht,
und X für O, N oder S steht.

2. Verbindungen nach Anspruch 1, **dadurch gekennzeichnet dass** die gestrichelt gezeichnete Bindung abwesend ist und R₁ für -CHCH₃OCH₂CHOHCH₃ oder - CHCH₃OCHR'CH₂OH steht.

3. Verbindungen nach einem der Ansprüche 1 oder 2 in Form eines Diastereoisomers oder Enantiomers.

4. Verbindungen nach einem der Ansprüche 1 oder 2 in Form einer Mischung von Diastereoisomeren.

5. Verbindungen nach Anspruch 1 oder 2 in Form einer razemischen Mischung.

6. Verfahren zur Herstellung einer Verbindung nach einem der Ansprüche 1 bis 5, **dadurch gekennzeichnet dass** die Ausgangsverbindung, oder eine Zwischenverbindung, eine Verbindung mit der folgenden Struktur ist:

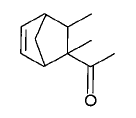

7. Verfahren zur Herstellung einer Verbindung nach einem der Ansprüche 1 bis 5, mit der Formel (I), in der R₁ -CHCH₃OCH₂CHOHR' oder -CHCH₃OCHR'CH₂OH ist und in welchen R' H, Me oder Et ist, **dadurch gekennzeichnet dass** die Ausgangverbindung oder eine Zwischenverbindung eine Verbindung mit der folgenden Struktur ist:

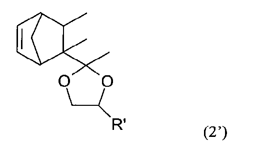

8. Verwendung einer Verbindung nach einem der Ansprüche 1 bis 5 als parfümierender Inhaltsstoff zur Herstellung einer parfümierenden zusammensetzung oder eines parfumierten Artikels.

9. Parfümierte Produkte, insbesondere Parfums, Toilettenwasser oder Kosmetika, wie Seifen, Duschgels, Badezusätze, Shampoos, Haarpflegemittel, Haushaltsprodukte, Waschmittel, Weichspüler, Deodorants oder Luftverbesserer, die eine Verbindung nach einem der Ansprüche 1 bis 5 oder eine Verbindung, hergestellt nach einem der Ansprüche 6 und 7, enthalten.
